(19) Europäisches Patentamt
European Patent Office
Office européen des brevets

(11) **EP 2 924 586 A1**

(12) **DEMANDE DE BREVET EUROPEEN**

(43) Date de publication:
**30.09.2015 Bulletin 2015/40**

(21) Numéro de dépôt: **15161211.6**

(22) Date de dépôt: **24.08.2012**

(51) Int Cl.:
*G06F 17/16* (2006.01)  *A61B 5/055* (2006.01)
*A61B 5/026* (2006.01)  *A61B 5/0275* (2006.01)
*G01R 33/48* (2006.01)  *G01R 33/563* (2006.01)
*G06T 7/00* (2006.01)  *A61B 6/00* (2006.01)
*A61B 5/00* (2006.01)  *G06F 17/12* (2006.01)

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **26.08.2011 FR 1157578**

(62) Numéro(s) de document de la (des) demande(s)
initiale(s) en application de l'article 76 CBE:
**12756792.3 / 2 758 895**

(71) Demandeur: **Olea Medical
13600 La Ciotat (FR)**

(72) Inventeur: **Pautot, Fabrice
13600 LA CIOTAT (FR)**

(74) Mandataire: **Brun, Philippe Alexandre Georges
MED'iNVENT CONSULTING
Espace Mistral - Bât.A
297 avenue du Mistral
ZI ATHELIA IV
13705 La Ciotat Cedex (FR)**

Remarques:
Cette demande a été déposée le 26.03.2015 comme
demande divisionnaire de la demande mentionnée
sous le code INID 62.

(54) **Système et procédé pour estimer une quantité d'intérêt d'un système dynamique artère/tissu/
veine**

(57) L'invention concerne un système et un procédé
pour estimer une quantité d'intérêt d'un système dynamique artère/tissu/veine d'un volume élémentaire - dit
voxel - d'un organe à partir d'images médicales selon les
méthodes dites oSVD ou cSVD. L'invention propose
deux modes de réalisation qui se distinguent par le temps
d'exécution nécessaire pour produire l'estimation de ladite quantité d'intérêt et permettent un diagnostic thérapeutique y compris en situation d'urgence.

EP 2 924 586 A1

Fig. 8

**Description**

L'invention concerne un système et un procédé pour estimer des paramètres hémodynamiques à partir d'images médicales. L'invention se distingue notamment des procédés connus par sa précision et selon le mode de réalisation par une rapidité d'exécution indispensable pour permettre un diagnostic thérapeutique en situation d'urgence.

L'invention s'appuie notamment sur des techniques d'imagerie de Perfusion par Résonance Magnétique (*Perfusion Weighted Magnetic Resonance Imaging* - PW-MRI - selon une terminologie anglo-saxonne) ou par Tomodensitométrie (*Computed Tomography* - CT - selon une terminologie anglo-saxonne). Ces techniques permettent d'obtenir des informations précieuses sur l'hémodynamique d'organes tels que le cerveau ou le coeur. Ces informations sont particulièrement cruciales pour un praticien cherchant à établir un diagnostic et à prendre une décision thérapeutique dans le traitement en hyper-urgence de pathologies telles que les accidents vasculaires cérébraux.

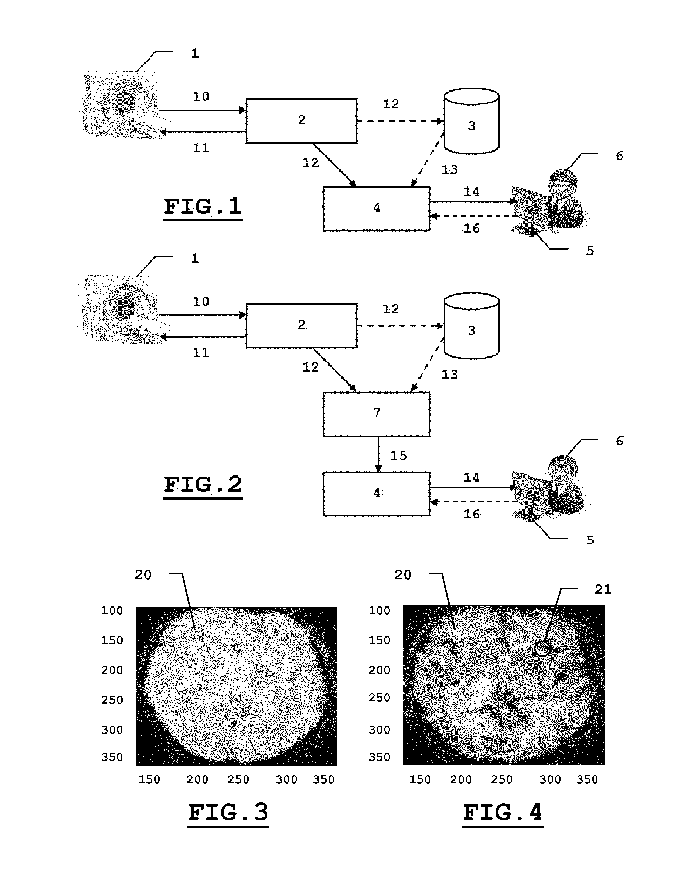

Pour mettre en oeuvre de telles techniques, on utilise un système d'imagerie médicale tel qu'illustré à titre d'exemple par les figures 1 et 2 exploitant un appareil 1 d'imagerie par Résonance Magnétique Nucléaire ou par Tomodensitométrie. Celui-ci délivre une pluralité de séquences d'images numériques 12 d'une partie du corps, notamment du cerveau. Ledit appareil applique pour cela une combinaison d'ondes électromagnétiques à haute fréquence sur la partie du corps considérée et mesure le signal réémis par certains atomes. L'appareil permet ainsi de déterminer la composition chimique et donc la nature des tissus biologiques en chaque point (ou voxel) du volume imagé.

Des séquences d'images sont analysées au moyen d'une unité de traitement dédiée 4. Cette unité de traitement délivre in fine à un praticien 6, une estimation des paramètres hémodynamiques à partir des images de perfusion, au moyen d'une interface homme-machine 5 adaptée. Le praticien peut ainsi réaliser un diagnostic et décider de l'action thérapeutique qu'il jugera adéquate.

Des images de perfusion par Résonance Magnétique Nucléaire ou par Tomodensitométrie sont obtenues en injectant un agent de contraste (par exemple un sel de gadolinium pour l'Imagerie par Résonance Magnétique) par voie intraveineuse et en enregistrant son bol au cours du temps au niveau de chaque voxel de l'image. Par souci de concision, nous omettrons les indices *x,y,z* pour identifier des voxels. Par exemple, au lieu de noter $S_{xyz}(t)$ le signal pour un voxel de coordonnées x,y,z, nous le noterons simplement $S(t)$. Il est entendu que les opérations et les calculs décrits dans la suite sont généralement effectués pour chaque voxel d'intérêt, de sorte à obtenir au final des images ou des cartes représentatives des paramètres hémodynamiques que l'on cherche à estimer.

Un modèle standard permet de relier l'intensité des signaux $S(t)$ mesurée au cours du temps *t* à la concentration $C(t)$ dudit agent de contraste.

Par exemple, en Tomodensitométrie de Perfusion, le signal pour chaque voxel est supposé directement proportionnel à la concentration : $S(t)=k \cdot C(t)+S_0$. En Imagerie de Perfusion par Résonance Magnétique Nucléaire, on suppose par exemple qu'il existe une relation exponentielle $S(t)=S_0 \cdot e^{-k \cdot TE \cdot C(t)}$. Dans les deux cas, $S_0$ représente l'intensité moyenne du signal avant l'arrivée de l'agent de contraste. Dans le cas de l'imagerie par Résonance magnétique nucléaire, *k* est une constante dépendant de la relation entre la susceptibilité paramagnétique et la concentration de l'agent de contraste dans le tissu et *TE* est le temps d'écho *(echo time* selon une terminologie anglo-saxonne). La valeur de la constante *k* pour chaque voxel étant inconnue, celle-ci est fixée à une valeur arbitraire pour tous les voxels d'intérêt. On obtient ainsi des estimations relatives et non pas absolues. Ces informations relatives restent toutefois pertinentes puisque l'on est intéressé principalement par la variation relative de ces valeurs dans l'espace, en particulier entre les tissus sains et les tissus pathologiques.

Dans toute la suite nous supposerons les signaux d'intensité expérimentaux $S(t)$ préalablement convertis en courbes de concentration *C(t).* Par exemple, dans le cas de l'imagerie de perfusion par résonance magnétique, nous

avons $C(t) = -\ln \dfrac{S(t)}{S_0}$, $S_0$ étant estimée en prenant, par exemple, la moyenne de $S(t)$ avant l'arrivée de l'agent de contraste.

La conservation de la masse de l'agent de contraste dans le volume de tissu contenu dans chaque voxel à chaque instant s'écrit $\dfrac{dC(t)}{dt} = BF \cdot \left[ C_a(t) - C_v(t) \right]$. $C_a(t)$ est la concentration de l'agent de contraste dans l'artère alimentant le volume de tissu (fonction d'entrée

artérielle ou *Arterial Input Fonction* - AIF - selon une terminologie anglo-saxonne). *BF* est le flux sanguin dans le volume de tissu *(Blood Flow* selon une terminologie anglo-saxonne) et $C_v(t)$ est la concentration de l'agent de contraste dans la veine drainant le volume de tissu (fonction de sortie veineuse ou *Venous Output Function* - VOF - selon une terminologie anglo-saxonne).

En supposant le système dynamique artère/tissu/veine linéaire et invariant dans le temps, on peut écrire $C_v(t) = C_a(t) \otimes h(t)$ où *h(t)* est la réponse impulsionnelle du système - ou encore fonction densité de probabilité du temps de transit de l'agent de contraste dans le tissu - et $\otimes$ désigne le produit de convolution. Une solution formelle de l'équation différentielle précédente avec condition initiale $C(t=0)=0$ s'écrit alors $C(t)=BF \cdot C_a(t) \otimes R(t)$ où $R(t)$ est la fonction de répartition complémentaire du temps de transit dans le volume de tissu (*residue function* selon une terminologie anglo-saxonne) définie par $R(t) = H(t) - \int_0^t h(\tau) d\tau$ où $H$ est la fonction généralisée créneau de Heaviside. A partir de la réponse impulsionnelle et de la fonction de répartition complémentaire, on définit un nouveau paramètre hémodynamique, le temps de transit moyen dans le tissu (*Mean Transit Time* - MTT - selon une terminologie anglo-saxonne) :

$$MTT = \int_0^{+\infty} t \cdot h(t) dt = \int_0^{+\infty} R(t) dt \qquad (\text{si } \lim_{t \to \infty} t \cdot h(t) = 0)$$

On peut définir également le volume sanguin dans le volume tissi (*Blood Volume* ou *BV* selon une terminologie anglo-saxonne) par la relation $BV=BF \cdot MTT$.

Si l'AIF utilisé est en retard d'un délai $\tau$ par rapport au véritable AIF, nous avons $C(t)=BF \cdot C_a(t-\tau) \otimes R(t)=BF \cdot C_a(t) \otimes R(t-\tau)$.

Ainsi le délai $\tau$ peut-être estimé en pratique comme l'instant où la fonction de répartition complémentaire estimée atteint son maximun (Time to Maximum - *TMAX* - suivant une terminologie anglo-saxonne).

Afin d'estimer les paramètres hémodynamiques tels que *BF*, *MTT*, *BV* ou *TMAX* ainsi que la fonction de répartition complémentaire R(t), il est donc nécessaire de déconvoluer les courbes de concentration $C(t)$ par des fonctions d'entrées artérielles $C_a(t)$ que l'on supposera données par la suite.

Pour effectuer cette opération de déconvolution de $C(t)$ par $C_{a(t)}$, le modèle standard de convolution $C(t)=BF \cdot C_a(t) \otimes R(t)$ est tout d'abord discrétisé temporellement aux instants $t_1,...,t_N$ d'échantillonnage du signale $S(t)$, en approchant numériquement l'intégrale de convolution $C_a(t) \otimes R(t)$. Sans perte de généralité, nous supposerons dans la suite l'échantillonnage périodique, de période $\Delta t=t_i-t_{i-1}$.

Par exemple, l'approximation de l'intégrale de convolution par la méthode des rectangles donne

$$\forall i = 1, N, \quad C(t_i) = BF \cdot \int_0^{t_i} C_a(\tau) \cdot R(t-\tau) d\tau \approx BF \cdot \Delta t \cdot \sum_{k=0}^{i} C_a(t_i) \cdot R(t_i - t_k)$$

On se ramène ainsi à un système linéaire $Ad = c$ de dimension $N$ en posant

$$A = \Delta t \cdot \begin{pmatrix} C_a(t_1) & 0 & ... & 0 \\ C_a(t_2) & C_a(t_1) & \ddots & \vdots \\ \vdots & \vdots & \ddots & 0 \\ C_a(t_N) & C_a(t_{N-1}) & ... & C_a(t_1) \end{pmatrix}, \quad b = \begin{vmatrix} R(t_1) \\ R(t_2) \\ ... \\ R(t_N) \end{vmatrix}, \quad c = \begin{vmatrix} C(t_1) \\ C(t_2) \\ ... \\ C(t_N) \end{vmatrix}$$

et $d = BF \cdot b$.

La matrice de Toeplitz triangulaire basse $A$ est très mal conditionnée et presque singulière, de sorte qu'on ne peut pas inverser numériquement ce système linéaire sous peine d'obtenir des solutions dénuées de sens et des estimations des paramètres hémodynamiques aberrantes. Il faut donc recourir à diverses méthodes pour obtenir par exemple une pseudo-inverse $\tilde{A}^{-1}$ de la matrice $A$ et par suite une estimation $\hat{d}$ de $d$ par $\tilde{d} = \tilde{A}^{-1} \cdot c$.

Parmi les nombreuses méthodes d'obtention d'une pseudo-inverse pour la matrice $A$, on trouve les méthodes classiques non paramétriques basées sur la troncature des valeurs singulières de $A$ telles que les méthodes sSVD (*Simple Singular Value Decomposition,*-selon une terminologie anglo-saxonne), cSVD (*Circular Singular Value Decomposition*) et oSVD (*Oscillation Index Singular Value Decomposition*).

La méthode sSVD a l'avantage d'être simple et rapide. Néanmoins, elle souffre de deux inconvénients majeurs :

- Elle est sensible au délai temporel $\tau$ entre la fonction d'entrée artérielle $C_a(t)$ et la courbe de concentration $C(t)$, c'est-à-dire qu'elle fournit des estimations des paramètres tels que *BF* et *MTT* qui dépendent de $\tau$ alors qu'elles ne le devraient pas ;

• En particulier, elle fournit des estimations des paramètres aberrantes lorsque ledit délai $\tau$ est négatif, c'est-à-dire lorsque la fonction d'entrée artérielle $C_a(t)$ est en retard par rapport à la courbe de concentration C(t).

Ces inconvénients sont palliés par les méthodes cSVD et oSVD qui sont par construction insensibles au délai $\tau$ et permettent de prendre en compte les délais négatifs.

La méthode cSVD (ainsi que la méthode sSVD) souffre néanmoins d'un inconvénient important : elle n'est pas adaptative : la régularisation est déterminée une fois pour toute à l'aide d'un paramètre *PSVD* propre à l'algorithme qu'on peut interpréter comme la fréquence de coupure d'un filtre passe-bas. Or la régularisation devrait au contraire s'adapter à chaque courbe de concentration expérimentale C(t), en particulier à leur rapport signal-sur-bruit. En pratique, on devrait donc préalablement déterminer une valeur du paramètre *PSVD* adaptée à chaque jeu de données de perfusion d'intérêt en déterminant par exemple la valeur permettant d'optimiser un critère donné (e.g. l'erreur relative sur un paramètre, etc.). Or cela est *stricto sensu* impossible puisque les valeurs théoriques des paramètres ne sont pas connues. D'autre part, une telle calibration pour chaque modalité de perfusion (e.g. CT Perfusion ou MR PWI), chaque appareil de mesure, chaque jeu des paramètres d'acquisition et même chaque type de signaux de perfusion (e.g. perfusion dans la matière blanche, dans la matière grise, perfusion saine ou pathologique) n'est clairement pas souhaitable. En pratique, ces calibrations sont rarement effectuées et le paramètre *PSVD* est souvent fixé de manière assez arbitraire.

La méthode oSVD permet de pallier en partie cet inconvénient en introduisant une régularisation semi-adaptative la rendant moins sensible aux différentes conditions expérimentales et aux différents types de signaux de perfusion. Cette méthode est par construction invariante par délai et semi-adaptative. En conséquence, la méthode oSVD est sensée fournir des estimations des paramètres hémodynamiques de meilleure qualité et plus robustes vis-à-vis des conditions opératoires que ne le font les méthodes cSVD et sSVD.

En revanche, la mise en oeuvre d'une telle méthode est complexe et non suffisamment documentée - comme en témoigne, à titre d'exemple et de manière imparfaite ou erronée, le document WO2005/009204A2 - pour rendre celle-ci applicable efficacement en milieu hospitalier. Il en est de même pour la méthode cSVD. oSVD et cSVD restent à ce jour théoriques. En outre, comme nous le verrons plus loin, la complexité algorithmique de la oSVD peut être de l'ordre de *N* fois celle de la cSVD et de 4*N* fois celle de la sSVD. Si les temps de calcul pour des jeux de données typiques d'imagerie de perfusion avec des moyens de calcul matériels conventionnels (i.e. ordinateur personnel ou station de travail) sont de l'ordre de quelques secondes pour les méthodes sSVD et cSVD, ils peuvent donc atteindre plusieurs minutes avec la méthode oSVD. Cela est parfaitement rédhibitoire en situation d'hyper-urgence clinique, comme par exemple lors du traitement des accidents vasculaires cérébraux où l'on estime que 4 millions de neurones meurent chaque minute.

La présente invention consiste selon un premier objet à implémenter la méthode oSVD pour pouvoir l'exploiter dans un système d'imagerie médicale. L'invention porte également sur un mode de réalisation particulièrement optimisé du point de vue du faible temps nécessaire à l'estimation d'une quantité d'intérêt. L'invention est illustrée préférentiellement - mais de manière non limitative - en lien avec l'exploitation d'un système d'imagerie médicale pour estimer une ou plusieurs quantités d'intérêts (ou paramètres(s) hémodynamique(s) par imagerie de perfusion. Le mode d'implémentation optimisée procure une complexité algorithmique du même ordre que celle de la méthode cSVD. On obtient dès lors des temps de calcul pour la oSVD conforme à l'invention de l'ordre de quelques secondes avec des moyens de calcul matériels conventionnels, acceptables en situation d'hyper-urgence clinique.

Selon un second objet, l'invention permet d'appliquer une approche équivalente pour favoriser la mise en en oeuvre de la méthode CSVD.

A cette fin, il est prévu un premier mode de réalisation d'un procédé mis en oeuvre par une unité de traitement d'un système d'analyse d'imagerie médicale pour produire une estimation d'un paramètre hémodynamique à partir de signaux d'intensité expérimentaux S(t), ladite estimation étant réalisée à partir d'une estimation $\hat{d}$ d'une quantité d'intérêt *d* d'un système dynamique artère/tissu/veine d'un volume élémentaire - dit voxel - d'un organe. Ladite estimation consiste à calculer $\hat{d} = \tilde{A}^{-1} \cdot c$, $\tilde{A}^{-1}$ étant une pseudo-inverse d'une matrice de convolution *A* et c décrivant une concentration d'un agent de contraste dans ledit voxel, ladite courbe de concentration résultant d'une conversion préalable desdits signaux expérimentaux. Selon ce premier mode de réalisation, le procédé comporte :

- une étape pour décomposer canoniquement *A* en valeurs singulières sous la forme $A = U \cdot S \cdot V^T$ où $S \equiv diag(\sigma_1, ..., \sigma_L)$ est la matrice diagonale des valeurs singulières classées par ordre croissant, $V^T$ est la transposée d'une matrice *V*, $V = (v_{ij})$ et $U = (u_{ij})$ sont deux matrices carrées de dimension $L \times L$ unitaires et réelles, $L \geq N$, *N* étant un nombre d'échantillons déterminé, $\hat{d}$, *A* et c étant respectivement de dimensions $L \times 1$, $L \times L$ et $L \times 1$ ;

- au moins une étape pour :

  o élaborer une pseudo-inverse $\tilde{A_l}^{-1}$ -1 de *A*, sous la forme $\tilde{A_l}^{-1} = V \cdot W_l \cdot U^T$, $U^T$ désignant la transposée de *U*, avec

$$W_l = diag\left(\underbrace{0,...,0}_{l},1/\sigma_{l+1},...,1/\sigma_L\right) \quad 0 < l \le L \; ;$$

o produire $\tilde{d_l} = \tilde{A_l}^{-1} \cdot c \quad ;$

- au moins une étape pour produire l'estimation du paramètre hémodynamique à partir de l'estimation de la quantité d'intérêt $\hat{d} = d_{l_F}$ produite à l'itération $l_F$, $l_F$ étant positif et inférieur ou égal à *L*.

L'invention prévoit une variante pour améliorer les performances d'exécution d'un tel procédé puisque $\tilde{A_l}^{-1}$ ne dépend pas des courbes de concentration. Ainsi pour tout voxel $V_j$ d'intérêt, l'étape itérative pour produire $\tilde{A_l}^{-1}$, $0 < l \le L$, peut être réalisée une fois pour toute après l'étape où *A* est décomposée canoniquement en valeurs singulières.

L'invention prévoit avantageusement que le procédé puisse comporter préalablement à chaque étape pour produire $d_l$ la vérification d'une condition de régularisation, ledit procédé s'interrompant à l'itération $l_F$ dès que ladite condition est satisfaite, l'estimation de *d* étant $\hat{d} = d_{l_F}$. A titre d'exemple préféré, la condition de régularisation peut consister en le calcul d'un index d'oscillation $OI_l$, ladite condition de régularisation étant satisfaite dès que $OI_{l_F} \le POI$, *POI* étant un seul prédéterminé.

Afin de répondre aux situations d'urgences cliniques, l'invention prévoit un second mode de réalisation optimisée du point de vue du temps de calcul pour produire pour produire une estimation d'un paramètre hémodynamique à partir de signaux d'intensité expérimentaux *S(t)*, ladite estimation étant réalisée à partir d'une estimation $\hat{d}$ d'une quantité d'intérêt *d* d'un système dynamique artère/tissu/veine d'un volume élémentaire - dit voxel - d'un organe. Au même titre que le procédé précédent, celui-ci est mis en oeuvre par une unité de traitement d'un système d'analyse d'imagerie médicale, ladite estimation consistant à calculer $\hat{d} = \tilde{A}^{-1} \cdot c$, $\tilde{A}^{-1}$ étant une pseudo-inverse d'une matrice de convolution *A* et c décrivant une concentration d'un agent de contraste dans ledit voxel, ladite courbe de concentration résultant d'une conversion préalable desdits signaux expérimentaux. Selon l'invention, le procédé comporte toujours une première étape pour décomposer canoniquement *A* en valeurs singulières sous la forme $A = U \cdot S \cdot V^T$ où $S = diag(\sigma_1,...,\sigma_L)$ est la matrice diagonale des valeurs singulières positives classées par ordre croissant, $V^T$ est la transposée d'une matrice *V*, $V = (v_{ij})$ et $U = (u_{ij})$ sont deux matrices carrées de dimension $L \times L$ unitaires et réelles, $L \ge N$, *N* étant un nombre d'échantillons déterminé, $\hat{d}$, *A* et c étant respectivement de dimensions $L \times 1$, $L \times L$ et $L \times 1$.

En revanche, un tel procédé compote en outre :

- préalablement à chaque étape pour produire $d_l$ la vérification d'une condition de régularisation consistant en le calcul d'un index d'oscillation $OI_l$, ladite condition de régularisation étant satisfaite dès que $OI_{l_F} \le POI$, *POI* étant un seul prédéterminé, ledit procédé s'interrompant à l'itération $l_F$ dès que ladite condition est satisfaite ;

- une étape pour produire l'estimation du paramètre hémodynamique à partir de l'estimation de la quantité d'intérêt $\hat{d} = d_{l_F}$ produite à l'itération $l_F$, $l_F$ étant strictement positif et inférieur ou égal à *L*.

Un tel index d'oscillation peut être calculé tel que

$$OI_l \equiv \frac{1}{L} \frac{1}{\max_{i=1,L} d_l(i)} \sum_{i=3}^{L} |d_l(i) - 2d_l(i-1) + d_l(i-2)| \quad \text{ou en variante, tel que}$$

$$OI_l \equiv \frac{1}{\Delta t^2 \cdot L} \frac{1}{\max_{i=1,L} d_l(i)} \sum_{i=3}^{L} |d_l(i) - 2d_l(i-1) + d_l(i-2)| \quad \text{où } \Delta t \text{ est la période d'échantillonnage des signaux}$$

*S(t)*.

Quel que soit le mode de réalisation conforme à l'invention choisi, pour initialiser l'étape itérative, un procédé conforme à l'invention peut comporter une étape initiale pour produire $d_0 = A_{l=0}^{-1} \cdot c$.

La matrice de convolution *A* peut être avantageusement bloc-circulante de dimension $L \times L$ définie par

$$A = \Delta t \cdot \begin{pmatrix} C_a(t_1) & 0 & \cdots & C_a(t_N) & \cdots & C_a(t_2) \\ \vdots & C_a(t_1) & & & \ddots & \vdots \\ C_a(t_N) & & \ddots & & & C_a(t_N) \\ 0 & \ddots & & \ddots & & 0 \\ \vdots & \ddots & \ddots & & \ddots & \vdots \\ 0 & \cdots & 0 & C_a(t_N) & \cdots & C_a(t_1) \end{pmatrix}$$

, $\Delta t$ étant la période d'échantillonnage, $C_a(t)$ étant une concentration de l'agent de contraste.

Pour mettre en oeuvre un procédé conforme à l'invention, celle-ci prévoit également une unité de traitement comportant des moyens de mémorisation, des moyens pour communiquer avec le monde extérieur et des moyens de traitement. Les moyens pour communiquer sont aptes à recevoir du monde extérieur une donnée de expérimentale c décrivant une courbe de concentration d'un agent de contraste dans un volume élémentaire - dit voxel - d'un organe et les moyens de traitement sont adaptés pour mettre en oeuvre un procédé pour produire une estimation d'un paramètre hémodynamique selon l'invention.

Préférentiellement, les moyens pour communiquer d'une telle unité de traitement peuvent délivrer une quantité d'intérêt estimée $\hat{d}$ selon un format approprié à une interface homme-machine apte à la restituer à un utilisateur.

Si le procédé mis en oeuvre permet conformément à l'invention de produire l'estimation d'un paramètre hémodynamique, une telle unité de traitement peut être adaptée pour délivrer - selon un format approprié - ladite estimation à une interface homme-machine apte à la restituer à un utilisateur.

L'invention concerne également tout système d'analyse d'imagerie médicale qui comporte une unité de traitement adaptée conformément à l'invention et une interface homme-machine apte à restituer à un utilisateur une quantité estimée selon un procédé conforme à l'invention et mis en oeuvre par ladite unité de traitement.

D'autres caractéristiques et avantages apparaîtront plus clairement à la lecture de la description qui suit et à l'examen des figures qui l'accompagnent parmi lesquelles :

- les figures 1 et 2, partiellement précédemment décrites, présentent deux variantes de réalisation d'un système d'analyse d'imagerie médicale ;
- Les figures 3 et 4 présentent respectivement une image de perfusion, obtenue par un appareil d'imagerie par Résonance Magnétique Nucléaire, d'une tranche d'un cerveau humain avant l'injection d'un agent de contraste et pendant la circulation de celui-ci dans les tissus dudit cerveau ;
- les figures 5a et 5b présentent un signal de perfusion $S(t)$ par Résonance Magnétique Nucléaire typique relatif à un voxel d'un cerveau humain ;
- la figure 6 présente une courbe de concentration $C(t)$ typique d'un agent de contraste circulant au sein d'un voxel d'un cerveau humain;
- la figure 7 présente une fonction d'entrée artérielle $C_a(t)$ typique ;
- les figures 8 et 9 présentent des procédés conformes à l'invention pour mettre en oeuvre la méthode oSVD ;
- la figure 8b présente un procédé conforme à l'invention pour implémenter la méthode cSVD ;
- les figures 10 et 11 présentent respectivement un exemple de carte relative à des quantités d'intérêt estimées conformément à l'invention.

De manière plus détaillée, la figure 1 permet de présenter un système d'analyse d'images médicales. Un appareil 1 d'imagerie par Résonance Magnétique Nucléaire ou par Tomodensitométrie est commandé à l'aide d'une console 2. Un utilisateur peut ainsi choisir des paramètres 11 pour piloter l'appareil 1. A partir d'informations 10 produites par l'appareil 1, on obtient une pluralité de séquences d'images numériques 12 d'une partie d'un corps d'un humain ou d'un animal. A titre d'exemple préféré, nous illustrerons les solutions issues de l'art antérieur ainsi que l'invention à l'aide d'images numériques issues de l'observation d'un cerveau humain. D'autres organes pourraient aussi être considérés.

Les séquences d'images 12 peuvent optionnellement être stockées au sein d'un serveur 3 et constituer un dossier médical 13 d'un patient. Un tel dossier 13 peut comprendre des images de différents types, telles que des images de perfusion ou de diffusion. Les séquences d'images 12 sont analysées au moyen d'une unité de traitement 4 dédiée. Ladite unité de traitement comporte des moyens pour communiquer avec le monde extérieur pour recueillir les images. Lesdits moyens pour communiquer permettent en outre à l'unité de traitement de délivrer in fine à un praticien 6 ou à un chercheur, une estimation de paramètres hémodynamiques 14 à partir des images de perfusion 12, au moyen d'une interface homme-machine adaptée 5. L'utilisateur 6 du système d'analyse peut ainsi confirmer ou infirmer un diagnostic, décider une action thérapeutique qu'il jugera adéquate, approfondir des travaux de recherche... Optionnellement, cet utilisateur peut paramétrer le fonctionnement de l'unité de traitement 4 au moyen de paramètres 16. Par exemple, il peut ainsi définir des seuils d'affichage ou choisir les paramètres estimés qu'il souhaite visualiser.

La figure 2 illustre une variante de réalisation d'un système d'analyse pour lequel une unité de prétraitement 7 analyse des séquences d'images 12 pour en déduire des données de perfusion 15 par voxel. L'unité de traitement 4 chargée d'estimer les paramètres hémodynamiques 14 est ainsi déchargée de cette action et met en oeuvre un procédé d'estimation à partir de données de perfusion 15 réceptionnés par ses moyens pour communiquer avec le monde extérieur.

La figure 3 illustre un exemple d'image typique 12 d'une tranche de 5 millimètres d'épaisseur d'un cerveau humain. Cette image est obtenue par Résonance Magnétique Nucléaire. A l'aide de cette technique, on peut obtenir, pour chaque tranche, une matrice de 128 x 128 voxels dont les dimensions sont de 1,5 x 1,5 x 5 millimètres. A l'aide d'une interpolation bilinéaire on peut produire une image à plat de 458 x 458 pixels telle que l'image 20.

La figure 4 illustre une image 20 similaire à celle présentée en liaison avec la figure 3. Toutefois cette image est obtenue après une injection d'un agent de contraste. Cette image est un exemple d'image de perfusion typique d'un cerveau. Les artères apparaissent ainsi clairement contrairement à la même image décrite en figure 3. Selon des techniques connues, il est possible de choisir une ou plusieurs fonctions d'entrée artérielle 21 dans l'hémisphère controlatérale à l'hémisphère pathologique pour estimer des paramètres hémodynamiques.

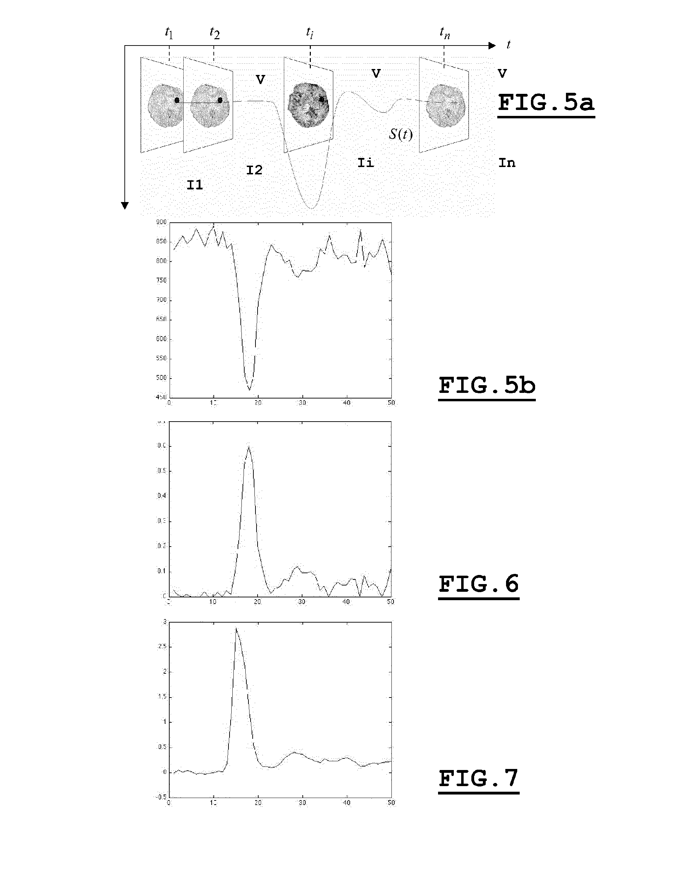

La figure 5b permet d'illustrer un exemple de signal de perfusion $S(t)$ par Résonance Magnétique Nucléaire tel que les données 15 délivrés par l'unité de prétraitement 7 décrite en liaison avec la figure 2. Le signal de perfusion est ainsi représentatif de l'évolution d'un voxel au cours du temps t à la suite d'une injection d'un agent de contraste. A titre d'exemple, la figure 5b décrit un tel signal sur une durée de 50 secondes. L'axe des ordonnées décrit l'intensité du signal dont l'unité est arbitraire. Pour obtenir un tel signal, l'unité de traitement 4 selon la figure 1 (ou en variante l'unité de prétraitement 7 selon la figure 2), analyse une séquence de n images de perfusion par Résonance Magnétique Nucléaire I1, I2, ..., Ii, ..., In à des instants $t_1$, $t_2$..., $t_i$..., $t_n$ comme le décrit, à titre d'exemple, la figure 5a. Pour un voxel donné, par exemple pour le voxel V, on détermine un signal de perfusion $S(t)$ représentatif de l'évolution du voxel au cours du temps t à la suite d'une injection d'un agent de contraste.

La figure 6 présente une courbe de concentration déduite d'un signal de perfusion tel que celui-ci décrit en figure 5b. Comme déjà évoqué précédemment, il existe une relation entre un signal de perfusion et une courbe de concentration associée. Ainsi, en Imagerie de Perfusion par Résonance Magnétique Nucléaire, il existe une relation exponentielle $S(t)=S_0 \cdot e^{-k.TE.C(t)}$ où $S_0$ est l'intensité moyenne du signal avant l'arrivée de l'agent de contraste, $TE$ est le temps d'écho (*echo time*) et $k$ est une constante dépendant de la relation entre la susceptibilité paramagnétique et la concentration de l'agent de contraste dans le tissu.

La figure 6 permet ainsi de visualiser au cours du temps, l'évolution de la concentration d'un agent de contraste au sein d'un voxel. On note un pic de forte amplitude lors du premier passage (*first pass,* en langue anglaise) de l'agent de contraste dans le voxel suivi de pics d'amplitudes plus faibles lié à un phénomène de recirculation (*second pass,* en langue anglaise) dudit agent de contraste.

La figure 7 illustre quant à elle, une fonction d'entrée artérielle typique $C_a(t)$ représentative de la circulation d'un agent de contraste au sein d'un voxel artériel tel que le voxel 21 présenté en liaison avec la figure 4. La figure 7 permet de constater notamment que le phénomène de recirculation après un premier passage de l'agent de contraste est très faible.

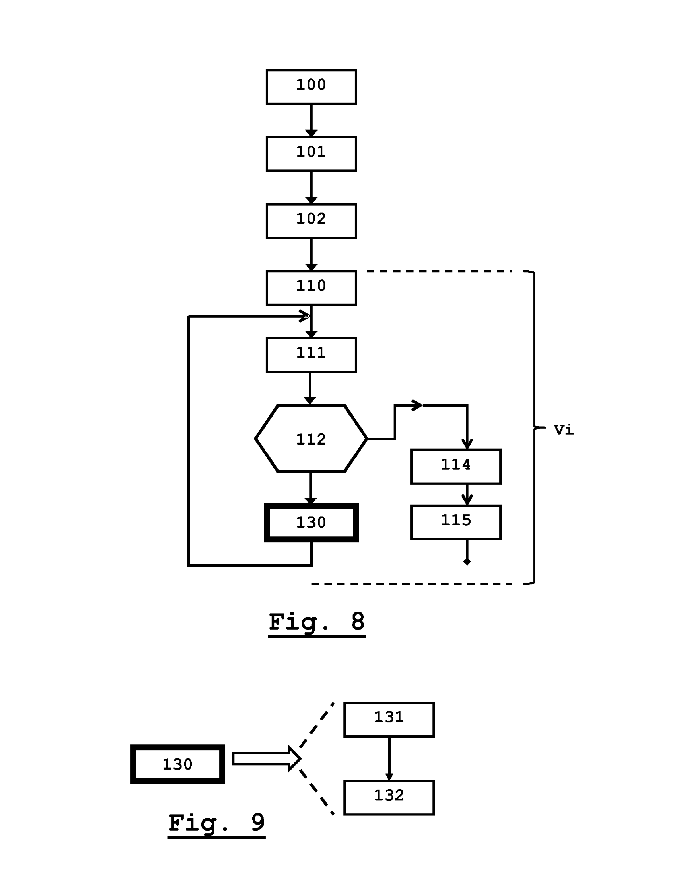

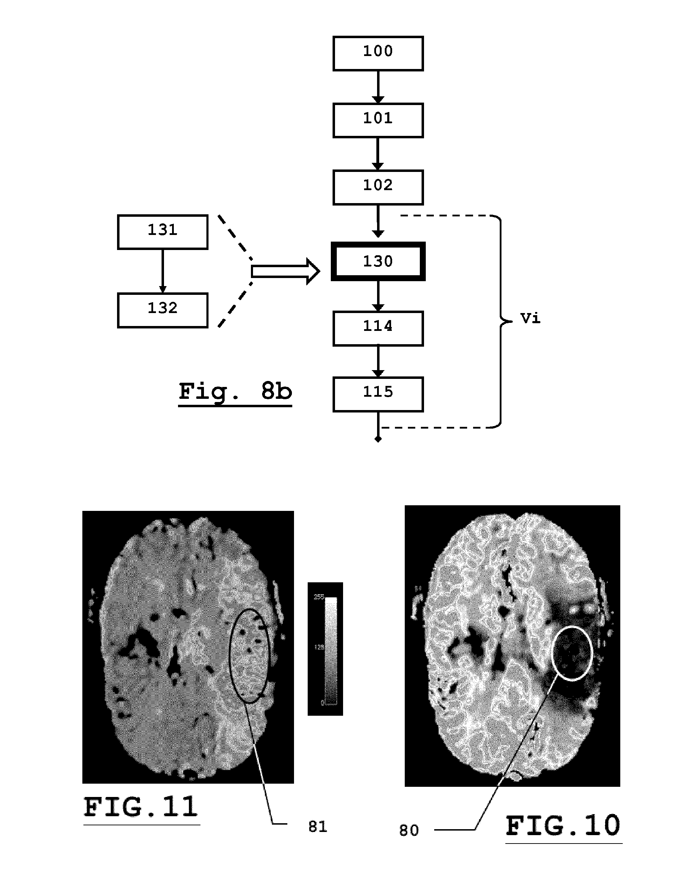

La figure 8 permet de décrire une implémentation conforme à l'invention de la méthode oSVD. Cette réalisation est appliquée préférentiellement à l'imagerie de perfusion.

Un procédé pour estimer une quantité d'intérêt peut comporter une première étape initiale 100 pour sélectionner une fonction d'entrée artérielle $C_a(t)$.

Selon la méthode oSVD, Le modèle standard de la perfusion est discrétisé temporellement aux instants de mesure sous la forme d'un système linéaire $c = A \cdot d$ avec:

$$A = \Delta t \cdot \begin{pmatrix} C_a(t_1) & 0 & \cdots & C_a(t_N) & \cdots & C_a(t_2) \\ \vdots & C_a(t_1) & & & \ddots & \vdots \\ C_a(t_N) & & \ddots & & & C_a(t_N) \\ 0 & \ddots & & \ddots & & 0 \\ \vdots & \ddots & \ddots & & \ddots & \vdots \\ 0 & \cdots & 0 & C_a(t_N) & \cdots & C_a(t_1) \end{pmatrix}, \quad b = \begin{vmatrix} R(t_1) \\ \vdots \\ R(t_N) \\ 0 \\ \vdots \\ 0 \end{vmatrix}, \quad c = \begin{vmatrix} C(t_1) \\ \vdots \\ C(t_N) \\ 0 \\ \vdots \\ 0 \end{vmatrix}$$

et $d=BF \cdot 6.$

*A, b, c et d* sont de dimensions respectives *L×L, L×1, L×1* et *L×1* avec $L \geq N$. Dans la suite, on note *v(i)*, *i=1,L*, les composantes d'un vecteur *v* de dimension *L*. Dans le cas où $L \geq 2N\text{-}1$, l'implémentation permet d'estimer des délais/*TMAX* aussi bien négatifs que positifs.

Une étape 101 consiste ainsi à construire la matrice de convolution circulaire bloc-circulante *A.*

Selon l'invention, la matrice de convolution circulaire bloc-circulante *A* est tout d'abord - en 102 - décomposée canoniquement en valeurs singulières sous la forme *A=U·S·V^T* où $S \equiv diag(\sigma_1,...,\sigma_L)$ est la matrice diagonale des valeurs singulières positives classées par exemple par ordre croissant (i.e. $\sigma_1 \leq \sigma_2 \leq ... \leq \sigma_L$), $U \equiv (u_{ij})$ et $V=(v_{ij})$ sont deux matrices carrées *L×L* unitaires réelles et *V^T* désigne ici la transposée de *V.*

Pour tout voxel *V_i* d'intérêt, un procédé conforme à l'invention consiste à mettre en oeuvre une étape itérative 130 pour produire une estimation $\hat{d}$ d'une quantité d'intérêt *d.* Cette production est préférentiellement réalisée tant qu'une condition de régularisation 112 n'est pas vérifiée. Pour cela, on calcule au préalable en 111 et à chaque itération *l*, un critère tel qu'un index d'oscillation *OI_l (oscillation index* OI, suivant une terminologie anglo-saxonne). A titre d'exemple préféré, ladite condition de régularisation est satisfaite dès que *OI_l* est supérieur ou égal à une valeur donnée *POI* prédéfinie strictement positive. L'itération de l'étape 130 s'arrête dès que ladite condition est satisfaite à l'itération *l_F.* Un tel procédé considère dès lors, en 114, que l'estimation $\hat{d}$ est *d_{l_F}.*

Selon l'invention, il devient possible de produire en 115 par exemple et respectivement des estimations des paramètres hémodynamiques *BF, MTT, BV,* ou encore du vecteur *b* par $\max\limits_{i=1,L} d_{l_F}(i)$ , $\dfrac{\Delta t}{\max\limits_{i=1,L} d_{l_F}(i)} \cdot \sum\limits_{i=1}^{L} d_{l_F}(i)$ ,

$$\Delta t \cdot \sum_{i=1}^{L} d_{l_F}(i) \quad \text{et} \quad \dfrac{d_{l_F}}{\max\limits_{i=1,L} d_{l_F}(i)}$$ · De même, selon l'invention il devient possible de produire des estimations de *TMAX,* par exemple, par $TMAX = \Delta t \cdot \arg\max\limits_{i=1,L} d_{l_F}(i)$ si *L=N* ou

$$TMAX = \begin{cases} \Delta t \cdot \arg\max\limits_{i=1,L} d_{l_F}(i) & \text{si} \quad \arg\max\limits_{i=1,L} d_{l_F}(i) < L/2 \\ -\Delta t \cdot \left( L - \arg\max\limits_{i=1,L} d_{l_F}(i) \right) & \text{si} \quad \arg\max\limits_{i=1,L} d_{l_F}(i) \geq L/2 \end{cases}$$

si $L \geq 2N\text{-}1$, où $\Delta t$ est la période d'échantillonnage des signaux *S(t).*

Pour mettre en oeuvre l'itération ou les itérations nécessaire(s) à la production de la quantité d'intérêt, l'invention prévoit une étape 110 pour initialiser tout paramètre nécessaire à ladite production.

Selon un premier mode de réalisation conforme à l'invention et décrit en liaison avec les figures 8 et 9, la production 130 de la quantité d'intérêt *d* consiste en deux sous-étapes 131 et 132. Ainsi on produit 131 à l'itération *l* une pseudo-inverse $\tilde{A}_l^{-1}$ de *A* sous la forme $\tilde{A}_l^{-1} = V \cdot W_l \cdot U^T$ où la matrice diagonale $W_l \equiv diag(w_1,...,w_L)$ est elle-même obtenue en procédant par itérations successives de la manière suivante. A la première itération (*l=0*) : $W_0 = S^{-1} = diag(1/\sigma_1,...,1/\sigma_L)$.

Lors d'une itération suivante et éventuelle (*l=1*), la matrice $W_1$ est obtenue en annulant le premier coefficient diagonal $w_1 = 1/\sigma_1$ de la matrice $W_0$ tel que $W_1 = diag(0,1/\sigma_2,...,1/\sigma_L)$ et ainsi de suite en annulant à chaque fois le coefficient diagonal $w_l$ de la matrice $W_{l-1}$ jusqu'à ce que la condition de régularisation $OI_{l_F} \leq POI$ soit remplie à l'itération *l = l_F.* On a donc $W \equiv W_{l*_F}$.

L'étape 131 permet de produire alors la pseudo-inverse $\tilde{A}_l^{-1} = V \cdot W_l \cdot U^T$ puis on calcule en 132 *d = BF·b* par $d_l = \tilde{A}_l^{-1} \cdot c$ .

Avantageusement l'index d'oscillation *OI_l* peut consister en :

$$OI_l \equiv \frac{1}{L}\frac{1}{\max\limits_{i=1,L} d_l(i)}\sum_{i=3}^{L}\left|d_l(i)-2d_l(i-1)+d_l(i-2)\right|$$

ou encore en :

$$OI_l \equiv \frac{1}{\Delta t^2 \cdot L}\frac{1}{\max\limits_{i=1,L} d_l(i)}\sum_{i=3}^{L}\left|d_l(i)-2d_l(i-1)+d_l(i-2)\right|$$

où $\Delta t$ est la période d'échantillonnage des signaux $S(t)$.

Ce premier mode de réalisation, que nous qualifierons de « direct » de la méthode oSVD, consiste donc à calculer le double produit matriciel $\tilde{A}_l^{-1}=V \cdot W_l^{-1} \cdot U^T$ puis le produit matrice-vecteur $d_l = \tilde{A}_l^{-1} \cdot c$ à chaque itération $l$ de l'algorithme, jusqu'à ce que la condition de régularisation $OI \leq POI$ soit remplie.

Avantageusement, il peut être prévu en variante de pré-calculer une fois pour toute toutes les pseudo-inverses $\tilde{A}_l^{-1}$, $l=0,L-1$ qui ne dépendent que de la fonction d'entrée artérielle et non pas des courbes de concentration. Selon cette variante, l'étape 131 est réalisée préalablement à toutes celles mises en oeuvre pour un voxel $V_i$ d'intérêt, par exemple à l'issue de l'étape 102 où $A$ est décomposée canoniquement en valeurs singulières.

Nous pouvons constater, y compris en implémentant cette variante qui permet de négliger l'impact de l'étape de pré-calcul des pseudo-inverses $\tilde{A}_l^{-1}$ par rapport au traitement de l'ensemble des voxels $V_i$ d'intérêt, qu'en considérant que le nombre $l_F$ d'itérations nécessaires pour remplir la condition de régularisation $OI_{l_F} \leq POI$ est de l'ordre de $N$, la mise en oeuvre de la méthode oSVD est donc d'une complexité algorithmique a priori de l'ordre de $O(L^2 \times N)=O(4N^3)$ pour chaque voxel d'intérêt, puisque les multiplications matrice-vecteur $d_l = \tilde{A}_l^{-1} \cdot c$ requièrent $O(L^2)$ opérations chacune.

En particulier, la complexité d'un tel procédé conforme à l'invention est de l'ordre de $N$ fois celle d'un algorithme pour mettre en oeuvre la cSVD et de $4N$ fois celle d'un algorithme pour mettre en oeuvre la sSVD. Le nombre de mesures $N$ étant typiquement compris entre 40 (e.g. en CT perfusion) et 70 (e.g. en MR PWI), l'algorithme oSVD est considérablement plus lent que les algorithmes cSVD et sSVD.

Pourtant, en tant qu'elle est invariante par délai et semi-adaptative, la méthode oSVD fournit des estimations des paramètres hémodynamiques de meilleure qualité et plus robustes vis-à-vis des conditions opératoires que ne le font les méthodes cSVD et sSVD. Elle leur est donc préférable si le temps de calcul n'est pas un critère primordial.

En situation d'urgence, un tel temps de mise en oeuvre pour produire une estimation d'une quantité d'intérêt peut être prohibitif.

On rappelle que le coût des moyens de calcul matériels conventionnels est typiquement proportionnel à leur puissance: une machine 50 fois plus puissante coûte typiquement 50 fois plus cher, de sorte qu'il n'est pas économiquement viable de s'en remettre à des moyens matériels conventionnels plus puissants afin d'obtenir des temps de calcul acceptables pour la méthode oSVD en situation d'hyper-urgence clinique. Certes, il est possible d'envisager des moyens de calcul matériels dédiés moins coûteux (e.g. cartes graphiques, cartes FPGA, *Field Programmable Gate Array* suivant une terminologie anglo-saxonne), mais par définition de telles solutions matérielles dédiées peuvent être coûteuses à développer et d'une applicabilité limitée.

La présente invention consiste en outre en une implémentation rapide de la méthode oSVD, d'une complexité algorithmique du même ordre que celle de la méthode cSVD. L'invention permet ainsi d'obtenir des temps de calcul pour la oSVD de l'ordre de quelques secondes avec des moyens de calcul matériels conventionnels, acceptables en situation d'hyper-urgence clinique. Un tel procédé conforme à un second mode de réalisation est illustré en liaison avec la figure 8. Un tel procédé reprend les étapes d'un procédé conforme au premier mode de réalisation. L'étape 130 est principalement différente. En 102, la matrice $A$ est toujours décomposée canoniquement en valeurs singulières sous la forme $A = U \cdot S \cdot V^T$ où $S \equiv diag(\sigma_1,...,\sigma_L)$ est la matrice diagonale des valeurs singulières positives. Celles-ci sont à présent avantageusement classées par ordre croissant (i.e. $\sigma_1 \leq \sigma_2 \leq ... \leq \sigma_L$).

Le principe de ce second mode de réalisation consiste à éviter de devoir pré-calculer (étape(s) 131 pour le premier mode de réalisation) les matrices $\tilde{A}_l^{-1}$ et surtout les produits matrice-vecteur $d_l = \tilde{A}_l^{-1} \cdot c$ (en 132 selon le précédent mode de réalisation) lors de chaque itération de l'algorithme. Voyons comment cela devient possible selon ce deuxième mode de réalisation.

A l'itération *l*>0 d'un procédé selon le premier mode de réalisation tel que décrit précédemment, nous avons donc $W_l = diag\left(\underbrace{0,...,0}_{l}, w_{l+1},..., w_L\right)$ de sorte que

$$W_l \cdot U^T = \begin{pmatrix} 0 & \cdots & & \cdots & 0 \\ \vdots & & & & \vdots \\ 0 & \cdots & & \cdots & 0 \\ w_{l+1}u_{1,l+1} & \cdots & & \cdots & w_{l+1}u_{L,l+1} \\ \vdots & & & & \vdots \\ w_L u_{1,L} & \cdots & & \cdots & w_L u_{L,L} \end{pmatrix} \begin{matrix} 1 \\ \vdots \\ l \\ l+1 \\ \vdots \\ L \end{matrix}$$

En notant $a_{ij}^l$ les coefficients de la matrice $\tilde{A}_l^{-1} = V \cdot W_l \cdot U^T$, nous avons donc par définition

$$a_{ij}^l = \sum_{k=l+1}^{L} v_{ik} w_k u_{jk} \, .$$

Le *i*-ème coefficient $d_l(i)$ du vecteur $d_l = \tilde{A}_l^{-1} \cdot c$ s'écrit alors

$$d_l(i) = \sum_{j=1}^{N} a_{ij}^l \cdot c_j = \sum_{j=1}^{N} c_j \cdot \sum_{k=l+1}^{L} v_{ik} w_k u_{jk}$$

puisque $c_j = 0$ si $N < j \le L$.

On peut alors écrire :

$$d_{l-1}(i) = \sum_{j=1}^{N} c_j \cdot \sum_{k=l}^{L} v_{ik} w_k u_{jk}$$

$$= \sum_{j=1}^{N} c_j \cdot \left( \sum_{k=l+1}^{L} v_{ik} w_k u_{jk} + v_{il} w_l u_{jl} \right) = d_l(i) + \sum_{j=1}^{N} c_j \cdot v_{il} w_l u_{jl}$$

de sorte à obtenir la formule de récurrence d'ordre un

$$d_l(i) = d_{l-1}(i) - \frac{v_{il}}{\sigma_l} \sum_{j=1}^{N} c_j \cdot u_{jl}$$

On constate donc que l'on peut exprimer directement le vecteur $d_l$ à partir du vecteur $d_{l-1}$ obtenu à l'itération précédente de l'algorithme oSVD sans avoir à effectuer une quelconque opération matricielle mais seulement en calculant une seule somme $\sum_{j=1}^{N} c_j \cdot u_{jl}$ .

Grâce à cette formule de récurrence mise en oeuvre à l'étape 130 d'un procédé conforme à celui décrit en liaison avec la figure 8, on passe d'une complexité algorithmique pour chaque voxel d'intérêt $V_i$ de l'ordre de $O(L^2 \times N)$ suivant l'implémentation directe à une complexité algorithmique de l'ordre de $O(L^2 + L \times N)$ puisqu'il faut initialiser - étape 110 - l'algorithme en calculant $d_0 = A^{-1} \cdot c$ de complexité $O(L^2)$.

Le coût supplémentaire en temps de calcul de cette implémentation par rapport à l'algorithme cSVD n'est donc plus que de l'ordre de $O(L \times N) = O(2N^2)$, à comparer à la complexité algorithmique de la cSVD elle-même en $O(L^2) = O(4N^2)$. En pratique, ce second mode d'implémentation rapide ou optimisée de la méthode oSVD est plus lente que l'algorithme cSVD seulement d'un facteur 2 ou 3 au lieu d'un facteur de l'ordre de $N$ suivant l'implémentation directe conforme au premier mode d'implémentation précédemment décrit.

Les calculs peuvent donc être effectués à nouveau en quelques secondes sur matériel conventionnel. Ainsi, l'invention rend possible l'usage de la méthode oSVD pour l'estimation de paramètres hémodynamiques par imagerie de perfusion (par exemple) sur moyens de calcul matériels conventionnels en situation d'hyper-urgence clinique.

Un procédé conforme à l'invention permet de mettre en oeuvre la méthode cSVD selon un mode opératoire similaire au premier mode de réalisation d'un procédé conforme à l'invention pour mettre en oeuvre la méthode oSVD. La figure 8b illustre un tel procédé appliqué à la méthode cSVD.

Ainsi, au même titre qu'un procédé conforme aux figures 8 et 9, un procédé conforme à l'invention pour mettre en oeuvre la méthode cSVD comporte une première étape 100 pour sélectionner une fonction d'entrée artérielle $C_a(t)$.

Selon la méthode cSVD, le modèle standard de la perfusion est également discrétisé temporellement aux instants de mesure sous la forme d'un système linéaire $c = A \cdot d$ avec:

$$A = \Delta t \cdot \begin{pmatrix} C_a(t_1) & 0 & \cdots & C_a(t_N) & \cdots & C_a(t_2) \\ \vdots & C_a(t_1) & & & \ddots & \vdots \\ C_a(t_N) & & \ddots & & & C_a(t_N) \\ 0 & \ddots & & \ddots & & 0 \\ \vdots & \ddots & \ddots & & \ddots & \vdots \\ 0 & \cdots & 0 & C_a(t_N) & \cdots & C_a(t_1) \end{pmatrix}, \quad b = \begin{vmatrix} R(t_1) \\ \vdots \\ R(t_N) \\ 0 \\ \vdots \\ 0 \end{vmatrix}, \quad c = \begin{vmatrix} C(t_1) \\ \vdots \\ C(t_N) \\ 0 \\ \vdots \\ 0 \end{vmatrix}$$

et $d = BF \cdot b$.

$A, b, c$ et $d$ sont de dimensions respectives $L \times L$, $L \times 1$, $L \times 1$ et $L \times 1$ avec $L \geq N$.

Une étape 101 consiste ainsi à construire la matrice de convolution circulaire bloc-circulante $A$.

Selon l'invention, la matrice de convolution circulaire bloc-circulante $A$ est tout d'abord - en 102 - décomposée canoniquement en valeurs singulières sous la forme $A = U \cdot S \cdot V^T$ où $S \equiv diag(\sigma_1, ..., \sigma_L)$ est la matrice diagonale des valeurs singulières positives classées par ordre croissant (i.e. $\sigma_1 \leq \sigma_2 \leq ... \leq \sigma_L$), $U \equiv (u_{ij})$ et $V \equiv (v_{ij})$ sont deux matrices carrées $L \times L$ unitaires réelles et $V^T$ désigne ici la transposée de $V$.

Pour tout voxel $V_i$ d'intérêt, un procédé conforme à l'invention consiste à mettre en oeuvre une étape 130 pour produire $d_{l_F}$ pour in fine produire l'estimation $\hat{d} = d_{l_F}$ en 114 d'une quantité d'intérêt $d$.

Dans le cas de la méthode cSVD, cette production de $d_{l_F}$ est préférentiellement réalisée en fixant une fois pour toute un entier $l_F$ tel que $0 \leq l_F < L$.

Selon l'invention, il devient possible de produire en 115 par exemple et respectivement des estimations des paramètres hémodynamiques $BF, MTT, BV$, ou encore du vecteur b par $\max_{i=1,L} d_{l_F}(i)$, $\dfrac{\Delta t}{\max_{i=1,L} d_{l_F}(i)} \cdot \sum_{i=1}^{L} d_{l_F}(i)$,

$\Delta t \cdot \sum_{i=1}^{L} d_{l_F}(i)$

et $\dfrac{d_{l_F}}{\max_{i=1,L} d_{l_F}(i)}$ .

L'invention décrite en liaison avec la figure 8, la production 130 de la quantité d'intérêt $d$ consiste en deux sous-étapes 131 et 132. Ainsi, dans le cas de la cSVD, on produit 131 une pseudo-inverse $\tilde{A}_{l_F}^{-1}$ de $A$ sous la forme

$$\tilde{A}_{l_F}^{-1} = V \cdot W_{l_F} \cdot U^T \text{ où la matrice diagonale } W_{l_F} \text{ est telle que } W_{l_F} = diag\left(\underbrace{0,...,0}_{l_F}, w_{l_F+1},..., w_L\right), \text{ l'entier } 0 < l_F$$

$< L$ étant fixé une fois pour toute par la méthode puis on calcule en 132 $d_{l_F} = BF \cdot b$ par $d_{l_F} = \tilde{A}_{l_F}^{-1} \cdot c$ .

En variante, l'invention prévoit en outre pouvoir de pré-calculer une fois pour toute toutes la pseudo-inverse $\tilde{A}_{l_F}^{-1}$ qui ne dépend que de la fonction d'entrée artérielle et non pas des courbes de concentration. Selon cette variante, l'étape 131 est réalisée préalablement à toutes celles mises en oeuvre pour un voxel $V_i$ d'intérêt, par exemple à l'issue de l'étape 102 où $A$ est décomposée canoniquement en valeurs singulières.

A titre d'exemple d'application, nous pouvons citer les principales étapes de mise en oeuvre de l'invention au moyen d'un système d'analyse d'imagerie médicale adapté, tel que celui décrit en figures 1 ou 2 :

- ouverture d'un dossier patient ou prise en compte de séquences d'images par l'unité de traitement 4 (ou de prétraitement 7) pour sélectionner des séquences d'images d'intérêt - en particulier, sélection des images I1 à In de perfusion au cours du temps à partir desquelles sont obtenus les signaux de perfusion $S(t)$ pour chaque voxel, tel qu'illustré en figure 5a ;
- prévisualisation au moyen d'une interface homme-machine 5 des images pour permettre à un utilisateur 6 d'identifier des tranches ou des zones d'intérêt ;
- configuration de l'unité de traitement 4 à partir des paramètres de configuration (informations introduite) pour permettre la mise en oeuvre du procédé d'estimation conforme à l'invention ;
- choix de la (ou les) quantité(s) d'intérêt à estimer ;
- estimation par l'unité de traitement 4 de quantités d'intérêt 14, tels que le flux sanguin $BF$ ou $MTT$ pour un organe tel que le cerveau humain ;
- délivrance desdites quantités d'intérêt estimées 14 à l'interface homme-machine 5 pour que celle-ci les présente *in fine* par exemple sous la forme de cartes où l'intensité ou la couleur de chaque pixel dépend de la valeur calculée pour en restituer la teneur au praticien.

L'invention prévoit donc d'afficher des estimations des paramètres sous forme de « cartes de paramètres » où l'intensité ou la couleur de chaque voxel dépend de la valeur calculée, par exemple de manière linéaire.

Les figures 10 et 11 permettent d'illustrer un mode d'affichage sous la forme de cartes, de certaines quantités d'intérêts tels que des paramètres hémodynamiques 14 estimés conformément à l'invention.

Ainsi, pour un cerveau humain analysé à l'aide d'imagerie par Résonance Magnétique Nucléaire, la figure 10 permet de visualiser une estimation des flux sanguins (blood flows, $BF$ suivant une terminologie anglo-saxonne). Elle présente une carte (458 x 458 pixels) relative à des flux sanguins cérébraux - en cas d'ischémie cérébrale - estimés conformément à l'invention. Une telle carte permet de mettre en évidence une zone ischémique probable 80.

La figure 11 permet d'illustrer une carte (458 x 458 pixels) relative à l'estimation des temps de transit moyen $MTT$. On peut constater en analysant la carte, une nette augmentation des $MTT$ dans le territoire 81 de l'artère cérébrale postérieure droite par rapport à l'hémisphère controlatérale consécutive à l'ischémie.

L'invention ne se limite pas uniquement aux méthodes oSVD ou cSVD proprement dites telles que décrites précédemment. Par exemple, l'invention s'applique tout aussi bien à des variantes de la méthode oSVD, par exemple à des méthodes comportant des critères d'arrêt différents du critère $OI \leq POI$ décrit précédemment.

De manière générale, l'invention s'applique à toute méthode de déconvolution numérique basée sur la troncature adaptative d'une décomposition en valeurs singulières d'une matrice de convolution. Par exemple, l'invention peut s'appliquer directement à toute méthode basée sur la matrice de convolution de Toeplitz triangulaire basse décrite précédemment au lieu de la matrice de convolution bloc-circulante des méthodes cSVD et oSVD. On obtiendrait ainsi par exemple une méthode rapide d'estimation des paramètres hémodynamiques par imagerie de perfusion qui serait à la sSVD ce que la oSVD est à la cSVD et qu'il conviendrait donc de dénommer méthode osSVD.

Enfin, l'invention ne s'applique pas seulement à l'imagerie de perfusion mais à tout type de données dont le traitement doit être effectué y compris en moindre temps.

**Revendications**

1. Procédé mis en oeuvre par une unité de traitement d'un système d'analyse d'imagerie médicale pour produire une estimation d'un paramètre hémodynamique (14) à partir de signaux d'intensité expérimentaux $S(t)$, ladite estimation étant réalisée à partir d'une estimation $\hat{d}$ d'une quantité d'intérêt $d$ d'un système dynamique artère/tissu/veine d'un volume élémentaire - dit voxel - d'un organe, ladite estimation consistant à calculer $\hat{d} = \tilde{A}^{-1} \cdot c$, $\tilde{A}^{-1}$ étant une pseudo-inverse d'une matrice de convolution $A$ et $c$ décrivant une courbe de concentration d'un agent de contraste dans ledit voxel, ladite courbe de concentration résultant d'une conversion préalable desdits signaux expérimentaux, **caractérisé en ce qu'**il comporte :

   - une étape (102) pour décomposer canoniquement $A$ en valeurs singulières sous la forme $A = U \cdot S \cdot V^T$ où $S \equiv diag(\sigma_1, ..., \sigma_L)$ est la matrice diagonale des valeurs singulières classées par ordre croissant, $V^T$ est la transposée d'une matrice $V$, $V = (v_{ij})$ et $U = (u_{ij})$ sont deux matrices carrées de dimension $L \times L$ unitaires et réelles, $L \geq N$, $N$ étant un nombre d'échantillons déterminé, $\hat{d}$, $A$ et $c$ étant respectivement de dimensions $L \times 1$, $L \times L$ et $L \times 1$;
   - au moins une étape itérative (130) pour :

      o élaborer (130, 131) une pseudo-inverse $\tilde{A_l}^{-1}$ de $A$, sous la forme $\tilde{A_l}^{-1} = V \cdot W_l \cdot U^T$, $U^T$ désignant la transposée de $U$, avec

$$W_l = diag\left( \underbrace{0, ..., 0}_{l}, 1/\sigma_{l+1}, ..., 1/\sigma_L \right) \quad 0 \leq l \leq L \quad ;$$

      o produire (130, 132) $d_l = \tilde{A_l}^{-1} \cdot c$ ;

   - une étape (115) pour produire l'estimation du paramètre hémodynamique (14) à partir de l'estimation de la quantité d'intérêt $\hat{d} = d_{l_F}$ produite à l'itération $l_F$, $l_F$ étant positif et inférieur ou égal à $L$.

2. Procédé selon la revendication précédente, selon lequel il comporte l'étape (131) pour produire $\tilde{A_l}^{-1}$, $0 < l \leq L$, réalisée une fois pour toute après l'étape (102) où $A$ est décomposée canoniquement en valeurs singulières.

3. Procédé selon l'une quelconque des revendications précédentes, pour lequel il comporte préalablement à chaque étape pour produire (130, 132) $d_l$ la vérification (112) d'une condition de régularisation, ledit procédé s'interrompant à l'itération $l_F$ dès que ladite condition est satisfaite, l'estimation (114) de $d$ étant $\hat{d} = d_{l_F}$.

4. Procédé selon la revendication précédente, pour lequel la condition de régularisation consiste en le calcul (111) d'un index d'oscillation $OI_l$, ladite condition de régularisation étant satisfaite dès que (112) $OI_{l_F} \leq POI$, $POI$ étant un seuil prédéterminé.

5. Procédé selon la revendication 3, selon lequel il comporte une étape (110) pour produire $d_0 = A_{l=0}^{-1} \cdot c$.

6. Procédé selon la revendication 4, pour lequel l'index d'oscillation est calculé tel que

$$OI_l \equiv \frac{1}{L} \frac{1}{\max\limits_{i=1,L} d_l(i)} \sum_{i=3}^{L} \left| d_l(i) - 2d_l(i-1) + d_l(i-2) \right| .$$

7. Procédé selon la revendication 4, pour lequel l'index d'oscillation est calculé tel que

$$OI_l \equiv \frac{1}{\Delta t^2 \cdot L} \frac{1}{\max\limits_{i=1,L} d_l(i)} \sum_{i=3}^{L} \left| d_l(i) - 2d_l(i-1) + d_l(i-2) \right| \quad \text{où } \Delta t \text{ est la période d'échantillonnage des si-}$$

gnaux $S(t)$.

**8.** Procédé selon l'une quelconque des revendications précédentes, selon lequel la matrice de convolution $A$ est bloc-circulante de dimension $L{\times}L$ définie (101) par

$$A = \Delta t \cdot \begin{pmatrix} C_a(t_1) & 0 & \cdots & C_a(t_N) & \cdots & C_a(t_2) \\ \vdots & C_a(t_1) & & & \ddots & \vdots \\ C_a(t_N) & & \ddots & & & C_a(t_N) \\ 0 & \ddots & & \ddots & & 0 \\ \vdots & \ddots & \ddots & & \ddots & \vdots \\ 0 & \cdots & 0 & C_a(t_N) & \cdots & C_a(t_1) \end{pmatrix},$$

$\Delta t$ étant la période d'échantillonnage, $C_a(t)$ étant une concentration de l'agent de contraste.

**9.** Unité de traitement (4) comportant des moyens de mémorisation, des moyens pour communiquer avec le monde extérieur et des moyens de traitement, **caractérisée en ce que** :

- les moyens pour communiquer sont aptes à recevoir du monde extérieur une donnée expérimentale c décrivant une courbe de concentration d'un agent de contraste dans un volume élémentaire - dit voxel - d'un organe ;
- les moyens de traitement sont adaptés pour mettre en oeuvre un procédé pour produire une estimation d'un paramètre hémodynamique selon l'une quelconque des revendications 1 à 8.

**10.** Unité de traitement selon la revendication précédente, pour laquelle les moyens pour communiquer délivrent l'estimation d'un paramètre hémodynamique selon un format approprié à une interface homme-machine (5) apte à la restituer à un utilisateur (6).

**11.** Système d'analyse d'imagerie médicale comportant une unité de traitement (4) selon la revendication précédente et une interface homme-machine (5) apte à restituer à un utilisateur (6) un paramètre hémodynamique estimé par ladite unité de traitement selon un procédé conforme à l'une quelconque des revendications 1 à 8.

**FIG.1**

**FIG.2**

**FIG.3**

**FIG.4**

**FIG.5a**

**FIG.5b**

**FIG.6**

**FIG.7**

**Fig. 8**

**Fig. 9**

**Fig. 8b**

**FIG.11**

**FIG.10**

**Europäisches Patentamt**
**European Patent Office**
**Office européen des brevets**

## RAPPORT DE RECHERCHE EUROPEENNE

Numéro de la demande

EP 15 16 1211

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | WO 2005/009204 A2 (GEN HOSPITAL CORP [US]; WU ONA [NL]; SORENSEN GREGORY [US]) 3 février 2005 (2005-02-03) * pages 1, 8-10, 13-16 * ----- | 1-11 | INV. G06F17/16 A61B5/055 A61B5/026 A61B5/0275 |
| A | ONA WU ET AL: "Tracer arrival timing-insensitive technique for estimating flow in MR perfusion-weighted imaging using singular value decomposition with a block-circulant deconvolution matrix", MAGNETIC RESONANCE IN MEDICINE, vol. 50, no. 1, juillet 2003 (2003-07), pages 164-174, XP055025384, ISSN: 0740-3194, DOI: 10.1002/mrm.10522 * page 2 * ----- | 1-11 | G01R33/48 G01R33/563 G06T7/00 A61B6/00 A61B5/00 G06F17/12 |
| A | US 2008/300484 A1 (WANG JING [US] ET AL) 4 décembre 2008 (2008-12-04) * alinéa [0028] - alinéa [0073] * ----- | 1-11 | |
| A | PETER GALL ET AL: "On the design of filters for fourier and oSVD-based deconvolution in bolus tracking perfusion MRI", MAGNETIC RESONANCE MATERIALS IN PHYSICS, BIOLOGY AND MEDICINE, CHAPMAN AND HALL, LONDON, GB, vol. 23, no. 3, 29 mai 2010 (2010-05-29), pages 187-195, XP019810040, ISSN: 1352-8661 * Section Material and Methods; page 188 - page 190 * ----- -/-- | 1-11 | DOMAINES TECHNIQUES RECHERCHES (IPC) A61B G01R G06F G06T |

Le présent rapport a été établi pour toutes les revendications

| Lieu de la recherche | Date d'achèvement de la recherche | Examinateur |
|---|---|---|
| Berlin | 29 juin 2015 | Huguet Serra, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

        .....................................................................................

& : membre de la même famille, document correspondant

EPO FORM 1503 03.82 (P04C02)

**EP 2 924 586 A1**

Europäisches Patentamt

European Patent Office

Office européen des brevets

# RAPPORT DE RECHERCHE EUROPEENNE

Numéro de la demande

EP 15 16 1211

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,P | TIMOTHÃ CR BOUTELIER ET AL: "Bayesian Hemodynamic Parameter Estimation by Bolus Tracking Perfusion Weighted Imaging", IEEE TRANSACTIONS ON MEDICAL IMAGING, IEEE SERVICE CENTER, PISCATAWAY, NJ, US, vol. 31, no. 7, juillet 2012 (2012-07), pages 1381-1395, XP011448909, ISSN: 0278-0062, DOI: 10.1109/TMI.2012.2189890 * le document en entier * ----- | 1-11 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Berlin | 29 juin 2015 | Huguet Serra, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 15 16 1211

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-06-2015

| Document brevet cité au rapport de recherche | Date de publication | Membre(s) de la famille de brevet(s) | Date de publication |
|---|---|---|---|
| WO 2005009204 A2 | 03-02-2005 | EP 1638447 A2<br>JP 2007525250 A<br>US 2007112264 A1<br>US 2010030071 A1<br>WO 2005009204 A2 | 29-03-2006<br>06-09-2007<br>17-05-2007<br>04-02-2010<br>03-02-2005 |
| US 2008300484 A1 | 04-12-2008 | AUCUN | |

EPO FORM P0460

**EP 2 924 586 A1**

## RÉFÉRENCES CITÉES DANS LA DESCRIPTION

**Documents brevets cités dans la description**

- WO 2005009204 A2